# EUROPÄISCHE PATENTSCHRIFT

(11) **EP 1 205 468 B1**
(45) Veröffentlichungstag und Bekanntmachung des Hinweises auf die Patenterteilung: **24.03.2004**
(21) Anmeldenummer: 01125354.9
(22) Anmeldetag: 29.10.2001
(51) Int. Cl.: C07C 205/06, C07C 201/08

(54) **Kontinuierliches isothermes Verfahren zur Herstellung von Mononitrotoluolen**
Continuous isothermal process for the preparation of mononitrotoluenes
Procédé continu isothermique pour la préparation de mononitrotoluènes

(30) Priorität: 08.11.2000 DE 10055359
(43) Veröffentlichungstag der Anmeldung: 15.05.2002
(73) Patentinhaber: Bayer Chemicals AG, 51368 Leverkusen (DE)
(72) Erfinder: Demuth, Ralf, Dr., 40724 Hilden (DE); Döbert, Frank, Dr., 50933 Köln (DE); Petersen, Harald, Dr., 41540 Dormagen (DE); Ronge, Georg, Dr., 40549 Düsseldorf (DE); Weber, Hans-Martin, Dr., 51373 Leverkusen (DE); Würminghausen, Thomas, Dr., 51381 Leverkusen (DE); Zirngiebl, Eberhard, Dr., 51061 Köln (DE)

(56) Entgegenhaltungen:
- EP-A- 0 156 199
- US-A- 2 256 999
- US-A- 5 648 565

## Beschreibung

Die vorliegende Erfindung betrifft ein kontinuierliches isothermes Verfahren zur Herstellung von Mononitrotoluolen unter Aufkonzentrierung der dabei anfallenden Abfallschwefelsäure und Rückführung der aufkonzentrierten Abfallschwefelsäure in das Verfahren.

Mononitrotoluole sind wichtige Zwischenprodukte für die Herstellung von optischen Aufhellern, Pflanzenschutzmitteln und Pharmaprodukten. Sie werden beispielsweise großtechnisch durch isotherme Nitrierung von Toluol hergestellt. Hierbei wird Toluol mit einer Mischung aus Schwefelsäure und Salpetersäure (Mischsäure, Nitriersäure) umgesetzt (s. beispielsweise Kirk-Othmer, Encyclopedia of Chemical Technology Vol. 17, 4^{th} Edition 1996, "Nitration" und "Nitrobenzenes and Nitrotoluene").

Bei der Umsetzung fällt ein erheblicher Anteil an mit organischen Verbindungen wie beispielsweise Dinitrotoluolen oder nitrierten Kresolen belasteter Abfallschwefelsäure an, der verfahrens- und kostenintensiv aufgearbeitet werden muss. Um den Anfall von Abfallsäure zu vermeiden, wurden Verfahren entwickelt, die eine Schwefelsäure-aufkonzentrierung beinhalten, wobei die aufkonzentrierte Schwefelsäure von Wasser und organischen Verbindungen befreit wird und anschließend in einem Kreislaufprozess wieder in die Nitrierreaktion zurückgeführt wird.

In DE 195 39 205 A werden Verfahrensparameter für die Mononitrierung von Aromaten offenbart, wobei die Mischsäuren so an die Eigenschaften des zu nitrierenden Aromaten angepasst werden, dass eine ca. 70 %ige Abfallschwefelsäure entsteht. Weiterhin ist der Einsatz von teilkonzentrierten Abfallsäuren mit einer Schwefelsäurekonzentration zwischen 85 % und 92 % beschrieben.

US-5 648 565 beschreibt ein Verfahren zur adiabatischen Herstellung von Mononitrotoluolen, indem man Toluol, Salpetersäure, Schwefelsäure und Wasser gleichzeitig oder sukzessive in ihrer Gesamtmenge intensiv miteinander vermischt und im Falle der kontinuierlichen Herstellung mindestens zweimal redispergiert, wozu eine Mischenergie pro Volumen des Reaktors von 1 bis 40 Watt pro Liter des gesamten Reaktionsgemisches, bevorzugt 3 bis 30 W/I, angewandt wird, und bei kontinuierlicher Durchführung die Rückvermischung weitgehend unterdrückt. In US 4,772,757 ist ein Verfahren zur Herstellung von Nitrobenzol beschrieben, wobei die anfallende Abfallsäure auf 75 bis 92 % aufkonzentriert und wieder in das Nitrierverfahren zurückgeführt wird. Da Toluol aufgrund der Methylgruppe im Vergleich zu Benzol deutlich oxidationsempfindlicher ist und bei der Nitrierung zur Bildung von Nebenprodukten neigt, ist bei der Übertragung von Reaktionsbedingungen zur Nitrierung von Benzol auf die Nitrierung von Toluol mit einer Erhöhung der Menge an unerwünschten Nebenprodukten zu rechnen.

Aufgrund der Löslichkeit von organischen Verbindungen in Schwefelsäure reichem sich in aufkonzentrierten Abfallsäuren, die in die Nitrierreaktion zurückgeführt werden, organische Nebenprodukte wie Oxalsäure oder Benzoesäure an. Weiterhin kann es zu einer Anreicherung an Nitrosylschwefelsäure kommen. Die Zersetzung dieser organischen Nebenprodukte und die dabei freiwerdende Zersetzungswärme führt zur unerwünschten Zersetzung des Reaktionsprodukts Nitrotoluol. Darüber hinaus ist eine Beeinträchtigung der Raum-Zeit-Ausbeute gegeben, da die organischen Nebenprodukte mit der eingesetzten Salpetersäure in oxidativen Abbaureaktionen reagieren können und somit ein Teil der Salpetersäure für die eigentliche Nitrierung nicht mehr zur Verfügung steht.

Um eine Anreicherung von organischen Nebenprodukten in der aufkonzentrierten Abfallschwefelsäure zu vermeiden, kann diese hochaufkonzentriert werden, d.h. auf 96 % aufkonzentriert werden. Unter diesen Aufkonzentrierungsbedingungen wird eine Zerstörung der organischen Nebenprodukte bewirkt. Nitrosylschwefelsäure kann durch Ausblasen mit Schwefeldioxid aus der aufkonzentrierten Abfallschwefelsäure entfernt werden.

Nachteilig an einer Aufkonzentrierung von Abfallschwefelsäure auf 96 % ist, dass diese bei Temperaturen von ca. 250°C durchgeführt werden muss, was bei großtechnischen Umsetzungen zu einem erhöhten Energieaufwand führt. Weiterhin muss die auf 96 % aufkonzentrierte Abfallschwefelsäure vor der Rückführung in die Nitrierreaktion in einem zusätzlichen Reaktionsschritt verdünnt werden, wobei die Verdünnung meist durch Mischen mit nicht-aufkonzentrierter Abfallsäure durchgeführt wird. Die Ausblasung von Nitrosylschwefelsäure muss in nachteiliger Weise in einem zusätzlichen Verfahrensschritt durchgeführt werden.

Es bestand daher Bedarf an einem kontinuierlichen isothermen Verfahren zur Herstellung von Mononitrotoluol, welches eine kostengünstige Aufkonzentrierung der Abfallschwefelsäure mit anschließender Rückführung in die Nitrierreaktion im Sinne eines Kreislaufprozesses erlaubt, ohne dass sich in der aufkonzentrierten Schwefelsäure organische Nebenprodukte anreichern.

Überraschenderweise wurde ein kontinuierliches Verfahren zur Herstellung von Mononitrotoluolen durch Umsetzung von Toluol mit Salpetersäure und Schwefelsäure unter isothermen Reaktionsbedingungen gefunden, wobei
a) 75 bis 93 %ige Schwefelsäure, 60 bis 70 %ige Salpetersäure und Toluol in einen Reaktor eingespeist werden,
b) am Reaktorausgang eine Trennung des Rohnitrotoluols von der Abfallschwefelsäure erfolgt
c) die Abfallsschwefelsäure in einer einstufigen Aufkonzentrierung auf 75 bis 93 % aufkonzentriert wird und
d) die aufkonzentrierte Abfallschwefelsäure in einer Kreisführung wieder in die Nitrierreaktion zurückgeführt wird.

Die im erfindungsgemäßen Verfahren vorliegende aufkonzentrierte Abfallschwefelsäure weist auch nach mehrmaliger Kreisführung keine Anreicherung von organischen Nebenprodukten wie Oxalsäure oder Benzoesäure oder anorganischen Verbindungen wie Nitrosylschwefelsäure auf. Darüber hinaus wird im erfindungsgemäßen Verfahren in vorteilhafter Weise eine verdünnte Salpetersäure eingesetzt, wodurch das Verfahren besonders kostengünstig betrieben werden kann. Trotz verhältnismäßig hoher Wassermengen, die durch den Einsatz von verdünnter Salpetersäure bedingt sind, läuft die Reaktion mit hohen Reaktionsgeschwindigkeiten ab.

Durch die Kreisführung der nach dem erfindungsgemäßen Verfahren aufkonzentrierten Abfallschwefelsäure wird überraschenderweise zusätzlich ein reaktionsbeschleunigender Effekt beobachtet.

Im erfindungsgemäßen Verfahren wird vorzugsweise eine 75 bis 93 %ige Schwefelsäure, besonders bevorzugt eine 84 bis 89 %ige Schwefelsäure, eingesetzt. Die eingesetzte Salpetersäure ist vorzugsweise eine 60 bis 70 %ige Salpetersäure, besonders bevorzugt wird eine 65 bis 68 %ige Salpetersäure eingesetzt. Der Anteil Salpetersäure im Schwefelsäure-Salpetersäuregemisch ergibt sich durch die Vorgabe der Konzentration der eingesetzten Schwefelsäure und Salpetersäure sowie der Konzentration der aufkonzentrierten Schwefelsäure und beträgt vorzugsweise ca. 15 bis 25 %, in einer besonders bevorzugten Ausführungsformen ca. 17 bis 20 %.

Im erfindungsgemäßen Verfahren werden vorzugsweise 0.98 bis 1.1 Äquivalente Toluol bezogen auf ein Äquivalent Salpetersäure eingesetzt, besonders bevorzugt werden 1.01 bis 1.05 Äquivalente Toluol bezogen auf ein Äquivalent Salpetersäure eingesetzt. Auch bei Toluolüberschuss bezogen auf Salpetersäure tritt im erfindungsgemäßen Verfahren überraschenderweise keine unter diesen Bedingungen sonst beobachtete Schwarzfärbung der Kreislauf-Abfallschwefelsäure (Black-Spent-Acid) auf.

Die im erfindungsgemäßen Verfahren eingesetzten Edukte Salpetersäure, Schwefelsäure, und Toluol werden vorzugsweise mit den in der Technik bekannten Mischorganen intensiv gemischt. Als Mischorgane können beispielsweise statische Mischer, Pumpen, Düsen, Rührer oder Kombinationen der genannten Mischorgane eingesetzt werden.

Das erfindungsgemäße Verfahren wird kontinuierlich unter isothermen Bedingungen in einem Reaktor durchgeführt. Als Reaktoren werden vorzugsweise kommerziell erhältliche Reaktoren wie beispielsweise Rohrreaktoren, Schlaufenreaktoren, Rührkessel oder auch Kombinationen von Schlaufenreaktoren und Rührkesseln eingesetzt.

In einer weiteren bevorzugten Ausführungsform wird das erfindungsgemäße Verfahren in mehrstufigen Reaktorkaskaden durchgeführt.

Das erfindungsgemäße Verfahren wird unter isothermen Bedingungen durchgeführt, wobei die Reaktionstemperatur vorzugsweise im Bereich von 20 bis 80°C, besonders bevorzugt im Bereich von 30 bis 70°C und ganz besonders bevorzugt im Bereich von 40 bis 65°C liegt.

Am Reaktorausgang beträgt die Schwefelsäurekonzentration in der Abfallschwefelsäure vorzugsweise 60 bis 80 %, besonders bevorzugt 65 bis 75 % und ganz besonders bevorzugt 69 bis 73 %. Die Abfallschwefelsäure ist nahezu frei von Salpetersäure und enthält neben Schwefelsäure Wasser, organische Verbindungen wie beispielsweise Dinitrotoluole oder nitrierte Kresole und gegebenenfalls salpetrige Säure.

Die Trennung des Rohnitrotoluols von der Abfallschwefelsäure wird vorzugsweise mit Hilfe eines statischen Scheiders oder eines Separators durchgeführt.

Durch die im erfindungsgemäßen Verfahren durchgeführte Aufkonzentrierung der Abfallschwefelsäure wird diese weitgehend von Wasser und organischen Verbindungen befreit, wobei die organischen Verbindungen entweder aus der Abfallschwefelsäure entfernt werden oder so zerstört werden, dass leichtflüchtige Verbindungen wie beispielsweise CO₂ entstehen, die aus der Abfallschwefelsäure ausgetragen werden.

Die einstufige Aufkonzentrierung wird vorzugsweise in einem Verdampfer durchgeführt. Um die erfindungsgemäße Konzentration 75 bis 93 %, bevorzugt 84 bis 89 %, zu erhalten, wird der Verdampfer vorzugsweise bei einem Druck von 50 bis 300 mbar, besonders bevorzugt von 60 bis 200 mbar und ganz besonders bevorzugt von 80 bis 150 mbar betrieben. Die Temperatur der Abfallschwefelsäure im Austritt des Verdampfers beträgt dabei vorzugsweise 100 bis 200°C, besonders bevorzugt 150 bis 190°C und ganz besonders bevorzugt 160 bis 180°C. Die Temperatur der ablaufenden aufkonzentrierten Abfallschwefelsäure wird vorzugsweise dazu genutzt, um in einem Gegenstromwärmetauscher die in den Verdampfer strömende Abfallschwefelsäure aufzuheizen. Hierbei wird die in den Verdampfer strömende Abfallschwefelsäure durch die Gegenstromführung vorzugsweise so weit aufgeheizt, dass diese bei Verdampferdruck überhitzt ist und somit ein Teil des Wassers und geringe Teile der Säure ohne zusätzliche Wärmezufuhr abdampfen (Flash-Verdampfung).

Für die einstufige Aufkonzentrierung im erfindungsgemäßen Verfahren wird vorzugsweise ein kommerziell erhältlicher, einstufiger kaskadierter Verdampfer mit Tantalrohrbündel verwendet, bei dem vom Eintritt kommend mit jeder Kaskade die Säurekonzentration erhöht wird, so dass in den ersten Kaskaden eine relativ niederkonzentrierte Säure vorliegt. Vorteil der niedrigen Konzentration in den ersten Kaskade ist einerseits, dass der Siedepunkt noch niedrig ist und somit eine hohe treibende Temperaturdifferenz für den Wärmeübergang vorliegt (kleinerer Verdampfer) und andererseits, dass bei niedrigen Säurekonzentrationen eventuell in der Abfallschwefelsäure vorliegende Nitrosylschwefelsäure aus der Reaktion gut entfernbar ist. Somit wird durch die Verwendung eins einstufigen kaskadierten Verdampfers im erfindungsgemäßen Verfahren das Ausblasen der Nitrosylschwefelsäure mit Schwefeldioxid und somit ein zusätzlicher Verfahrensschritt vermieden.

Vorzugsweise wird ein Abtriebteil eingesetzt, um eine besonders gute Reduzierung des Gehalts an organischen Verbindungen und/oder des Gehalts an Nitrosylschwefelsäure zu erreichen. Als Abtriebsteil wird ein mit Destillationseinbauten versehener Destillationskolonnenabschnitt bezeichnet, auf den von oben die bei Verdampferdruck flüssige oder etwas überhitzte Abfallschwefelsäure geleitet wird und der im Gegenstrom von unten mit dem aus dem Verdampfer aufsteigenden Dampf betrieben wird. Als Destillationseinbauten kommen im Abtriebsteil die dem Fachmann bekannten Kolonneneinbauten wie beispielsweise Böden, Packungen und Füllkörper zur Anwendung. In einer bevorzugten Ausführungsform werden druckverlustarme Destillationseinbauten wie geordnete Packungen oder Füllkörper eingesetzt. Die Verweilzeit im Abtriebsteil bei gleichzeitig geringer Säurekonzentration führt zusammen mit dem durch die Destillationseinbauten intensivierten Stoffaustausch in vorteilhafter Weise zu einer schnellen Zersetzung und Abtrennung von organischen und anorganischen Verbindungen.

Das im erfindungsgemäßen Verfahren erhaltene Rohnitrotoluol enthält in der Regel weniger als 0,5 % dinitrierte Verbindungen und weniger als 0,8 % Dinitrokresole.

### Beispiele

### Beispiel 1

In einer Miniplant-Anlage wurden einer Rührkesselkaskade pro Stunde 0,80 kg 87,7 %ige Schwefelsäure, 0,31 kg 67 %ige Salpetersäure und 0,32 kg Toluol zugeführt. Die Temperatur in den Rührkesseln betrug ca. 40°C. Nach Beendigung der Reaktion wurde das Rohnitrotoluol von der Abfallschwefelsäure mittels eines statischen Scheiders abgetrennt. Die Abfallschwefelsäure wurde über einen Vorwärmer einem Verdampfer zugeführt und bei 100 mbar und 168°C auf 87,7 % aufkonzentriert, wobei organische Verbindungen abdestilliert oder zerstört wurden. Die Abfallschwefelsäure wurde der Nitrierreaktion erneut zugeführt.

Das erhaltene Rohnitrotoluol wies folgende Zusammensetzung auf:
3,27 % Toluol, 57,58 % ortho-Nitrotoluol, 4,13 % meta-Nitrotoluol, 34,68 % para-Nitrotoluol, 0,08 % Dinitrotoluol und 0,38 % Dinitrokresol

### Beispiel 2

Einer Kaskade aus mehreren Schlaufenreaktoren wurden stündlich 3000 1 ca. 97 %iges Toluol, welches geringe Mengen nitrierter Toluole und Kresole enthielt, 3700 1 87 %ige Schwefelsäure und 1800 67 bis 68 %ige Salpetersäure zugeführt. Die Reaktoren wurden zwischen 43 und 47°C betrieben. Nach Beendigung der Reaktion wurde das Rohnitrotoluol von der Abfallschwefelsäure mittels eines Separators abgetrennt. Die Abfallschwefelsäure wurde bei ca. 170°C und 100 mbar wieder auf den Anfangswert von 87 % aufkonzentriert und in die Nitrierreaktion zurückgeführt.

Das erhaltene Rohnitrotoluol wies folgende Zusammensetzung auf:
4,13 % Toluol, 57,12 % ortho-Nitrotoluol, 4,18 % meta-Nitrotoluol, 34,17 % para-Nitrotoluol, 0,12 % Dinitrotoluol und 0,71 % Kresole.

### Vergleichsbeispiel 1

Einem Rohrreaktor wurden stündlich 188 kg 70 %ige Schwefelsäure, 8,1 kg 67 %ige Salpetersäure und 8,6 kg Toluol kontinuierlich zugeführt. Die Reaktion wurde unter adiabatischen Bedingungen durchgeführt. Nach der Abtrennung des Rohnitrotoluols wurde die Abfallschwefelsäure in einem Verdampfer bei 60 mbar und 90°C wieder auf die eingesetzte Ausgangskonzentration von 70 % aufkonzentriert. Die aufkonzentrierte Abfallschwefelsäure wurde erneut der Nitrierreaktion zugeführt.

Im Vergleich zu den erfindungsgemäßen Beispielen wird nach einigen Stunden eine deutliche Anreicherung von organischen Verbindungen (als Hauptkomponente Oxalsäure) in der Kreislaufsäure beobachtet.

### Vergleichsbeispiel 2

Einer kontinuierlich arbeitenden Reaktorkaskade wurden stündlich 2940 1 ca. 72 bis 73 %ige gebrauchte Schwefelsäure, 1830 1 98 %ige Salpetersäure und 4700 Toluol, welches ca. 3 % nitrierte Toluole und Kresole enthielt, zugeführt. Die Reaktion erfolgte bei 44 bis 54°C. Nach Abtrennung des Rohnitrotoluols wurde ein Teil der Abfallschwefelsäure erneut der Nitrierreaktion zugeführt, der Rest wurde einer zweistufigen Aufkonzentrierung auf 96 %ige Schwefelsäure unterzogen und ebenfalls wieder der Nitrierreaktion zugeführt.

Das erhaltene Rohnitrotoluol wies folgende Zusammensetzung auf:
4,48 % Toluol, 56,38 % ortho-Nitrotoluol, 4,28 % meta-Nitrotoluol, 34,20 % para-Nitrotoluol, 0,58 % Dinitrotoluol und 0,73 % Kresole.

Im Vergleich mit den erfindungsgemäßen Beispielen finden sich im erhaltenen Rohnitrotoluol deutlich höhere Mengen an unerwünschtem Dinitrotoluol.

## Patentansprüche

1. Kontinuierliches Verfahren zur Herstellung von Mononitrotoluolen durch Umsetzung von Toluol mit Salpetersäure und Schwefelsäure unter isothermen Reaktionsbedingungen, wobei
a) 75 bis 93 %ige Schwefelsäure, 60 bis 70 %ige Salpetersäure und Toluol in einen Reaktor eingespeist werden,
b) am Reaktorausgang eine Trennung des Rohnitrotoluols von der Abfallschwefelsäure erfolgt
c) die Abfallsschwefelsäure in einer einstufigen Aufkonzentrierung auf 75 bis 93 % aufkonzentriert wird und
d) die aufkonzentrierte Abfallschwefelsäure in einer Kreisführung wieder in die Nitrierreaktion zurückgeführt wird.

2. Verfahren gemäß Anspruch 1, **dadurch gekennzeichnet, dass** eine 84 bis 89 %ige Schwefelsäure eingesetzt wird.

3. Verfahren gemäß einem oder mehreren der vorangegangenen Ansprüche 1 und 2, **dadurch gekennzeichnet, dass** eine 65 bis 68 %ige Salpetersäure eingesetzt wird.

4. Verfahren gemäß einem oder mehreren der vorangegangenen Ansprüche 1 bis 3, **dadurch gekennzeichnet, dass** 0,98 bis 1,1 Äquivalente Toluol, bezogen auf ein Äquivalent Salpetersäure eingesetzt werden.

5. Verfahren gemäß einem oder mehreren der vorangegangenen Ansprüche 1 bis 3, **dadurch gekennzeichnet, dass** 1,01 bis 1,05 Äquivalente Toluol, bezogen auf ein Äquivalent Salpetersäure eingesetzt werden.

6. Verfahren gemäß einem oder mehreren der vorangegangenen Ansprüche 1 bis 5, **dadurch gekennzeichnet, dass** die Schwefelsäurekonzentration am Reaktorausgang 60 bis 80 % beträgt.

7. Verfahren gemäß einem oder mehreren der vorangegangenen Ansprüche 1 bis 6, **dadurch gekennzeichnet, dass** die Aufkonzentrierung in einem Verdampfer bei einem Druck von 60 bis 200 mbar und einer Temperatur von 100 bis 200°C durchgeführt wird.

8. Verfahren gemäß einem oder mehreren der vorangegangenen Ansprüche 1 bis 7, **dadurch gekennzeichnet, dass** die Aufkonzentrierung in einem kaskadierten Verdampfer durchgeführt wird.

9. Verfahren gemäß einem oder mehreren der vorangegangenen Ansprüche 7 und 8, **dadurch gekennzeichnet, dass** der Verdampfer mit einem Abtriebteil betrieben wird.

10. Verfahren gemäß einem oder mehreren der vorangegangenen Ansprüche 1 bis 9, **dadurch gekennzeichnet, dass** die Reaktionstemperatur im Bereich von 20 bis 80°C liegt.

## Claims

1. Continuous process for preparing mononitrotoluenes by reacting toluene with nitric acid and sulphuric acid under isothermal reaction conditions, in which
a) 75 to 93% strength sulphuric acid, 60 to 70% strength nitric acid and toluene are fed into a reactor,
b) the crude nitrotoluene is separated from the waste sulphuric acid at the reactor outlet
c) the waste sulphuric acid is concentrated in a single-stage concentration to 75 to 93% and
d) the concentrated waste sulphuric acid is recycled in a circulation back to the nitration reaction.

2. Process according to Claim 1, **characterized in that** an 84 to 89% strength sulphuric acid is used.

3. Process according to one or more of the preceding Claims 1 and 2 **characterized in that** a 65 to 68% strength nitric acid is used.

4. Process according to one or more of the preceding Claims 1 to 3,
**characterized in that** 0.98 to 1.1 equivalents of toluene, based on one equivalent of nitric acid, are used.

5. Process according to one or more of the preceding Claims 1 to 3, **characterized in that** 1.01 to 1.05 equivalents of toluene, based on one equivalent of nitric acid, are used.

6. Process according to one or more of the preceding Claims 1 to 5, **characterized in that** the sulphuric acid concentration at the reactor outlet is 60 to 80%.

7. Process according to one or more of the preceding Claims 1 to 6, **characterized in that** the concentration is carried out in an evaporator at a pressure of 60 to 200 mbar and a temperature of 100 to 200°C.

8. Process according to one or more of the preceding Claims 1 to 7, **characterized in that** the concentration is carried out in a cascade evaporator.

9. Process according to one or more of the preceding Claims 7 and 8, **characterized in that** the evaporator is operated with a stripping part.

10. Process according to one or more of the preceding Claims 1 to 9, **characterized in that** the reaction temperature is in the range from 20 to 80°C.

## Revendications

1. Procédé continu pour la préparation de mononitrotoluènes par réaction de toluène avec de l'acide nitrique et de l'acide sulfurique dans des conditions de réaction isothermes,
a) de l'acide sulfurique à de 75 à 93 %, de l'acide nitrique à de 60 à 70 % et du toluène étant introduits dans un réacteur,
b) une séparation du nitrotoluène brut de l'acide sulfurique usagé étant réalisée à la sortie du réacteur,
c) l'acide sulfurique usagé étant concentré dans une concentration en une étape à de 75 à 93 % et
d) l'acide sulfurique usagé concentré dans une circulation étant à nouveau renvoyé dans la réaction de nitruration.

2. Procédé selon la revendication 1, **caractérisé en ce que** l'on utilise un acide sulfurique à de 84 à 89 %.

3. Procédé selon l'une ou plusieurs quelconques des revendications 1 et 2 précédentes, **caractérisé en ce que** l'on utilise un acide nitrique à de 65 à 68 %.

4. Procédé selon l'une ou plusieurs quelconques des revendications 1 à 3 précédentes, **caractérisé en ce que** l'on utilise de 0,98 à 1,1 équivalent de toluène, rapporté à un équivalent d'acide nitrique.

5. Procédé selon l'une ou plusieurs quelconques des revendications 1 à 3 précédentes, **caractérisé en ce que** l'on utilise de 1,01 à 1,05 équivalent de toluène, rapporté à 1 équivalent d'acide nitrique.

6. Procédé selon l'une ou plusieurs quelconques des revendications 1 à 5 précédentes, **caractérisé en ce que** la concentration en acide sulfurique à la sortie du réacteur est comprise entre 60 et 80 %.

7. Procédé selon l'une ou plusieurs quelconques des revendications 1 à 6 précédentes, **caractérisé en ce que** l'on réalise la concentration dans un évaporateur à une pression de 60 à 200 mbar et à une température de 100 à 200°C.

8. Procédé selon l'une ou plusieurs quelconques des revendications 1 à 7 précédentes, **caractérisé en ce que** l'on réalise la concentration dans un évaporateur en cascade.

9. Procédé selon l'une ou plusieurs quelconques des revendications 7 et 8 précédentes, **caractérisé en ce que** l'évaporateur fonctionne avec une partie de rectification.

10. Procédé selon l'une ou plusieurs quelconques des revendications 1 à 9 précédentes, **caractérisé en ce que** la température de réaction se trouve dans le domaine de 20 à 80°C.
